(19) **Europäisches Patentamt**

**European Patent Office**

**Office européen des brevets**

(11) **EP 1 492 550 B1**

(12) **EUROPÄISCHE PATENTSCHRIFT**

(45) Veröffentlichungstag und Bekanntmachung des Hinweises auf die Patenterteilung:
**14.12.2005 Patentblatt 2005/50**

(51) Int Cl.$^7$: **A61K 35/78**, A61P 37/08, A61P 29/00

(21) Anmeldenummer: 03724971.1

(22) Anmeldetag: **07.04.2003**

(86) Internationale Anmeldenummer:
**PCT/EP2003/003605**

(87) Internationale Veröffentlichungsnummer:
**WO 2003/084554 (16.10.2003 Gazette 2003/42)**

(54) **WEIDENEXTRAKT**

WILLOW EXTRACT

EXTRAIT DE SAULE

(84) Benannte Vertragsstaaten:
**AT BE BG CH CY CZ DE DK EE ES FI FR GB GR HU IE IT LI LU MC NL PT RO SE SI SK TR**

(30) Priorität: **09.04.2002 DE 10215553**

(43) Veröffentlichungstag der Anmeldung:
**05.01.2005 Patentblatt 2005/01**

(73) Patentinhaber: **Dr. Willmar Schwabe GmbH & Co. 76227 Karlsruhe (DE)**

(72) Erfinder:
• **SCHMIDT, Peter, C.**
**72074 Tübingen (DE)**
• **LAUFER, Stefan**
**89143 Blaubeuren (DE)**
• **HEMPEL, Bernd**
**73730 Esslingen (DE)**
• **BERNDT, Dieter**
**73733 Esslingen (DE)**
• **WALKER, Roland**
**72074 Tübingen-Pfrondorf (DE)**
• **MAUZ, Matthias**
**70597 Stuttgart-Degerloch (DE)**

(74) Vertreter: **Reitstötter - Kinzebach Patentanwälte, Sternwartstrasse 4 81679 München (DE)**

(56) Entgegenhaltungen:
**DE-A- 19 615 578**

• **SCHMID B ET AL: "Pharmacokinetics of salicin after oral administration of a standardised willow bark extract." EUROPEAN JOURNAL OF CLINICAL PHARMACOLOGY. GERMANY AUG 2001, Bd. 57, Nr. 5, August 2001 (2001-08), Seiten 387-391, XP002248721 ISSN: 0031-6970**
• **TENA M T ET AL: "Superficial fluid extraction of t-resveratrol and other phenolics from a spiked solid." FRESENIUS' JOURNAL OF ANALYTICAL CHEMISTRY, Bd. 361, Nr. 2, Mai 1998 (1998-05), Seiten 143-148, XP002248722 ISSN: 0937-0633**
• **CHEMICAL ABSTRACTS, vol. 136, no. 12, 2002 Columbus, Ohio, US; abstract no. 180644w, ZHENG, SHANG-ZEN ET AL.: "CHEMICALCONSTITUENTS OF THE ESSENTIAL OIL OF THE LEAF OF SALIX MATSUDANA K. PREPARED BY SUPERCRITICAL CO2 FLUID EXTRACTION." Seite 432; XP002210355 & XIBEI SHIFAN DAXUE XUEBAO, ZIRAN KEXUEBAN, Bd. 37, Nr. 4, 2001, Seiten 77-81,**
• **CHRUBASIK S ET AL: "Effectiveness of willow bark extract in painful conditions" ZEITSCHRIFT FUR PHYTOTHERAPIE 2002 GERMANY, Bd. 23, Nr. 6, 2002, Seiten 263-266, XP009014413 ISSN: 0722-348X**

**Beschreibung**

Die vorliegende Erfindung betrifft einen Weidenextrakt mit verbesserter antiinflammatorischer Wirkung.

Wässrige und wässrig-alkoholische Extrakte von Pflanzenteilen der Weide (Salix L.) sind bekannt für ihre entzündungshemmenden Effekte. Sie werden daher in Arzneimitteln, aber auch in Kosmetikprodukten, beispielsweise zur Verringerung von Hautreizungen, eingesetzt.

So beschreibt die US 9,722,843 eine Creme zur Behandlung der Haut, die in einer Grundlage aus Lanolin einen Extrakt aus Zweigen der Trauerweide enthält. Die Creme bringt Erleichterung bei brennender und juckender Haut. Die DE 44 03 157 A wiederum beschreibt kosmetische und pharmazeutische Präparate zur topischen Anwendung, die schmerzlindernde und/oder schmerzstillende Eigenschaften besitzen und einen Extrakt von Salix alba oder Salix fragilis enthalten.

Die DE 196 15 578 beschreibt die Verwendung eines Salix nigra-Extrakts zur Behandlung und Prophylaxe irritativer, erythematöser und entzündlicher oder allergischer Erscheinungen.

Die US 6,254,899 beschreibt Zusammensetzungen, die einen Extrakt einer Pflanze der Gattung Salix und einen Extrakt einer Pflanze der Gattung Tanacetum enthält. Die Zusammensetzungen besitzen antiinflammatorische und analgetische Eigenschaften und sind zur therapeutischen und präventiven Behandlung von Rheuma, entzündlichen Erkrankungen, arterieller Hypertension, vaskularen Spasmen und Tachykardien brauchbar. Außerdem werden sie zur therapeutischen und präventiven Behandlung von Migräne eingesetzt.

Die DE 199 24 688 A beschreibt Salicylalkohol-Derivate, die von dem wichtigsten Inhaltsstoff der Weidenextrakte abgeleitet sind, nämlich dem Salicin. Diese Derivate inhibieren die Prostaglandinsynthese und sie besitzen daher antiinflammatorische, antipyretische, antiphlogistische oder analgetische Wirkung.

Weidenextrakte werden durch Extraktion von Pflanzenteilen der Weide, insbesondere der Rinde, mit wässrigem Ethanol gewonnen. Die wichtigste Verbindungsgruppe, die auch zur Hauptsache für die pharmakologische Wirkung verantwortlich ist, ist die Gruppe der Phenolglykoside, zu denen in erster Linie das Salicin und die Ester des Salicins zählen. Darüber hinaus sind in dem Extrakt auch zahlreiche weitere Stoffe enthalten, beispielsweise Flavonoide und Flavanverbindungen.

Den Extrakten des Standes der Technik ist gemeinsam, dass ihre pharmakologische Wirkung nicht zufriedenstellend ist.

Der vorliegenden Erfindung liegt daher die Aufgabe zugrunde, einen Weidenextrakt mit im Vergleich zum Stand der Technik verbesserter pharmakologischer Wirkung zur Verfügung zu stellen.

Eine weitere Aufgabe besteht darin, einen Weidenextrakt in einer Form zur Verfügung zu stellen, die eine leichte Verarbeitbarkeit des Extrakts zu pharmazeutischen Dosierungsformen, insbesondere oralen Dosierungsformen, ermöglicht.

Überraschenderweise wurde nun gefunden, dass diese Aufgaben gelöst werden, wenn man die Extraktion von Pflanzenteilen der Weide mit einem überkritischen Gas durchführt.

Gegenstand der vorliegenden Erfindung ist daher ein Verfahren zur Herstellung eines Weidenextraktes, insbesondere eines Weidenrindenextraktes, wobei man zerkleinerte, von einer Weide stammende Pflanzenteile mit einem Lösungsmittelgemisch aus einem überkritischen Gas und mindestens einem organischen Co-Solvens extrahiert und den erhaltenen Flüssigextrakt von dem Lösungsmittelgemisch befreit.

Die zu extrahierenden Pflanzenteile stammen von Pflanzen der Gattung Salix L., insbesondere von Pflanzen der Untergattung Salix und Vetrix DUM. Zu dieser Gattung gehörende Arten sind beispielsweise in Hagers Handbuch der pharmazeutischen Praxis, 5. Auflage, 1991, Band 2, Seite 469 ff. oder im Lexikon der Biologie, Herder-Verlag, 1987, Band 8, Stichwort Weide, aufgeführt. Dazu gehören beispielsweise Salix alba, Salix fragilis, Salix triandra, Salix pentandra, Salix nigra, Salix purpurea, Salix cinerea, Salix capria, Salix daphnoides, Salix nigricans und Salix babilonica. Bevorzugt verwendet man Pflanzenteile von Salix daphnoides, Salix babylonica (insbesondere aus China) und Salix alba.

Als Pflanzenteile verwendet man vorzugsweise die Zweige, insbesondere die Spitzen der Zweige und besonders bevorzugt die Rinde davon. Die Zweige und Zweigspitzen sind insbesondere ohne Blätter von Vorteil, d. h. wenn sie im Winter geerntet werden.

Zur Extraktion werden die Pflanzenteile in üblicher Weise zerkleinert, beispielsweise durch Mahlen. Gegebenenfalls werden die zerkleinerten Pflanzenteile vor der Extraktion getrocknet. Man trocknet im allgemeinen 5 bis 48 h bei 30 bis 50 °C.

Zur Extraktion wird ein Gemisch aus einem überkritischen Gas und mindestens einem organischen Co-Solvens verwendet. Bei den überkritischen Gasen handelt es sich um Gase, die bei realisierbaren Drücken und nicht so hohen Temperaturen den überkritischen Zustand erreichen können. Der überkritische Zustand wird bei Überschreiten des kritischen Punktes erreicht, welcher durch die kritische Temperatur und den kritischen Druck definiert ist. Geeignete überkritische Gase sind in Hagers Handbuch der pharmazeutischen Praxis, Band 2, 5. Auflage, 1991, Seite 1030, angegeben. Vorzugsweise verwendet man Kohlendioxid ($CO_2$) oder Distickstoffmonoxid ($N_2O$).

Geeignete organische Co-Solventien sind insbesondere wasserlösliche Lösungsmittel, wie Alkohole, beispielsweise Methanol, Ethanol, oder Isopropanol, Ketone, beispielsweise Aceton oder Methylethylketon, oder Ether, wie Tetrahydrofuran oder Dioxan. Die Menge an Co-Solvens liegt im allgemeinen im Bereich von 1 bis 50 Vol.-%, insbesondere 5 bis 30 Vol.-%, bezogen auf das Gemisch.

Vorzugsweise erfolgt die Extraktion bei einem Druck im Bereich von 80 bis 150 bar, insbesondere 90 bis 120 bar. Die Extraktionstemperatur liegt im allgemeinen im Bereich von 40 bis 100°C, insbesondere 50 bis 80 °C. Die Extraktionszeit liegt im allgemeinen im Bereich von 0,5 bis 10 Stunden.

Im allgemeinen wird das Lösungsmittelgemisch im Durchfluss durch die zu extrahierenden Pflanzenteile geschickt. Die Durchflussrate hängt dabei von den gewählten Extraktionsbedingungen ab und ist in weitem Bereich variierbar. Im allgemeinen wählt man die Durchflussrate so, dass die Menge an Lösungsmittelgemisch, die während der gesamten Extraktionszeit durch die zu extrahierenden Pflanzenteile fließt, im Bereich von 5 bis 100 Gew.-Teilen pro Gew.-Teil zu extrahierender Pflanzenteile liegt.

Der erhaltene Extrakt wird anschließend von dem Gemisch aus überkritischem Gas und Co-Solvens befreit. Dies erfolgt durch Entspannung in einer oder mehreren Stufen unter Abscheidung der extrahierten Stoffe. Die Entspannung erfolgt so, dass ein Druck eingestellt wird, der unterhalb des kritischen Drucks liegt (bei Kohlendioxid 73,8 bar und bei Distickstoffmonoxid 72,5 bar). Vorzugsweise liegt der Abscheidedruck im Bereich von 30 bis 50 bar. Im allgemeinen erfolgt die Entspannung bei einer Temperatur im Bereich von 40 bis 100 °C. Man erhält auf diese Weise einen Extrakt in fester Form, der je nach Entspannungsbedingungen, noch Reste des Co-Solvens und gegebenenfalls Wasser enthalten kann. Der auf diese Weise erhaltene Extrakt wird vorzugsweise einer Trocknung unterzogen. Im allgemeinen trocknet man bei einer Temperatur im Bereich von 30 bis 60 °C, insbesondere 35 bis 55 °C. Die Trocknungszeit liegt im allgemeinen im Bereich von 1 bis 100 Stunden. Vorzugsweise führt man eine Vakuumtrocknung oder eine Gefriertrocknung durch, was sich als besonders vorteilhaft erwiesen hat, weil der Extrakt dann in lockerer, pulvrig bis granulatartiger und fließfähiger Form erhalten wird und direkt, beispielsweise zu Tabletten, weiterverarbeitet werden kann. Weiter hat sich der erfindungsgemäße Extrakt als besonders stabil erwiesen. Er ist daher im Gegensatz zu den Extrakten des Standes der Technik, standardisierbar und leicht zu pharmazeutischen Dosierungsformen verarbeitbar.

Weiter hat sich gezeigt, dass der erfindungsgemäße Weidenextrakt einen Salicingehalt von wenigstens 25 Gew.-%, bezogen auf den Feststoffanteil, aufweist. Vorzugsweise liegt der Salicingehalt im Bereich von 30 bis 50 Gew.-% und insbesondere 35 bis 45 Gew.-%. Der Salicingehalt bezieht sich auf den Gehalt an Salicin, der nach alkalischer Verseifung des Extraktes vorliegt. Er wird bestimmt nach 60minütiger Behandlung einer methanolischen Lösung des Extraktes mit einem Überschuß an wässrigem Natrium- oder Kaliumhydroxid bei 60 °C. Im allgemeinem verwendet man 50 ml 0,1 N oder 25 ml 0,2 N Natrium- oder Kaliumhydroxidlösung pro g trockenem Extrakt. Die Bestimmung des Salicingehaltes in dem nach der Verseifung erhaltenen Gemisch erfolgt mittels HPLC (stationäre Phase: LiChrosorb Si 60 RP-18, 10 μm der Fa. Merck KgaA, 64271 Darmstadt, Deutschland; mobile Phase: Wasser mit 2,5 % V/V Tetrahydrofuran).

Der Salicingehalt des erfindungsgemäßen Extraktes ist damit wesentlich höher als der Salicingehalt eines durch Extraktion mit wässrigem Ethanol erhaltenen Extraktes, der im allgemeinen im Bereich von etwa 15 bis 18 Gew.-% liegt.

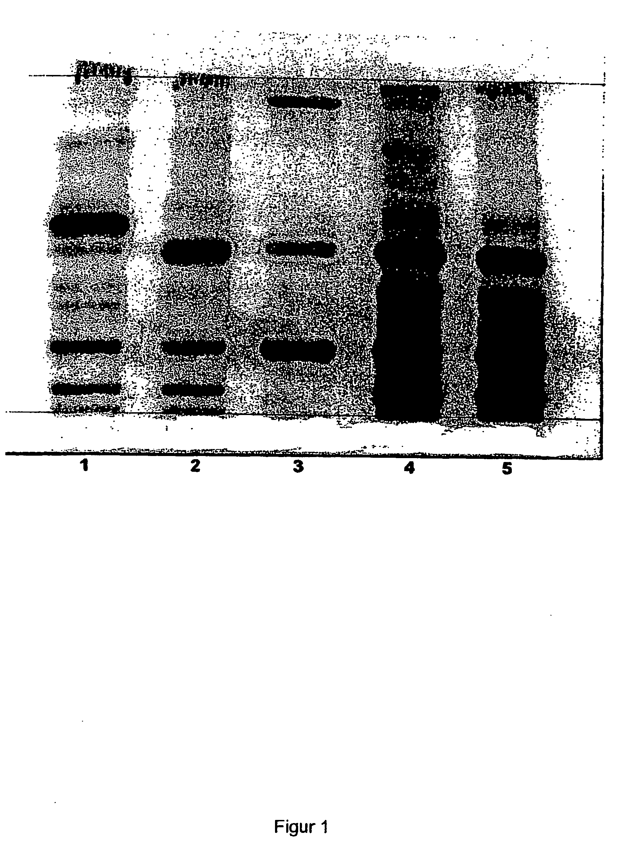

Die Unterschiede zum Stand der Technik lassen sich anhand der Dünnschichtchromatogramme zeigen, die in Figur 1 dargestellt sind. Reagenzien und Methodik zur Anfertigung der Dünnschichtchromatogramme sind in den Beispielen erläutert. Figur 1 enthält folgende Chromatogramme:

Chromatogramm 1:

Erhalten mit einer Probe eines erfindungsgemäßen Weidenextraktes.

Chromatogramm 2:

Erhalten mit einer Probe eines erfindungsgemäßen Weidenextraktes nach alkalischer Verseifung.

Chromatogramm 3:

Erhalten mit einer Vergleichslösung, die Glukose, Salicin und Cianidanol (im Chromatogramm von unten nach oben) enthält.

Chromatogramm 4:

Erhalten mit einer Probe eines Extraktes des Standes der Technik (Dragenopharm 1581).

Chromatogramm 5:

Erhalten mit einer Probe eines Extraktes des Standes der Technik (Dragenopharm 1581) nach alkalischer Verseifung.

Die Dünnschichtchromatogramme zeigen, dass sich der erfindungsgemäße Weidenextrakt von einem durch Extraktion mit wässrigem Ethanol gewonnenen Weidenextrakt des Standes der Technik unterscheidet. Der Unterschied kommt auch in der pharmakologischen Aktivität zum Ausdruck, wie das anhand der in den Beispielen angegebenen Messergebnisse ersichtlich ist.

Der erfindungsgemäße Extrakt besitzt antiinflammatorische, antipyretische, antiallergische und analgetische Wirkung. Darüber hinaus hat sich gezeigt, dass der Extrakt ein Cyclooxigenase-Hemmer ist und insbesondere die Cyclooxigenase-2 hemmt. Der erfindungsgemäße Extrakt ist daher bei der Behandlung von Erkrankungen, die mit einer Veränderung der Arachidonsäuremetabolisierung einhergehen, und insbesondere zur Behandlung von Erkrankungen des rheumatischen Formenkreises und zur Prävention von allergisch induzierten Erkrankungen sowie zur Behandlung dermatologischer Erkrankungen, wie Psoriasis, Urtikaria, akute und chronische Exantheme allergischer und nicht-allergischer Genese brauchbar.

Der erfindungsgemäße Extrakt wird im allgemeinen in Form eines pharmazeutischen Mittels dosiert und verabreicht, d. h. in Mischung mit geeigneten pharmazeutischen Trägern oder Verdünnungsmitteln. Der Extrakt kann oral oder topisch verabreicht werden.

Die Art des pharmazeutischen Mittels oder Trägers beziehungsweise des Verdünnungsmittels hängt von der gewünschten Verabreichungsform ab. Feste orale Mittel können beispielsweise als Tabletten, Kapseln, Dragees oder Pulver vorliegen und übliche Exzipienzien enthalten, wie Bindemittel (z. B. Sirup, Akazia, Gelatine, Sorbit, Tragant oder Polyvinylpyrrolidon), Füllstoffe (z. B. Laktose, Zucker, Maisstärke, Calciumphosphat, Sorbit oder Glycin), Gleitmittel (z. B. Magnesiumstearat, Talkum, Polyethylenglykol), Fließregulierungsmittel (Siliciumdioxid), desintegrierende Mittel (z. B. Stärke) oder Netzmittel (z. B. Natriumlaurylsulfat). Flüssige orale Mittel können beispielsweise in Form wässriger oder öliger Suspensionen, Lösungen, Emulsionen, Sirupe, Elixiere etc. vorliegen. Derartige flüssige Mittel können übliche Additive, beispielsweise Suspendiermittel, Geschmackstoffe, Verdünnungsmittel oder Emulgatoren, enthalten.

Topische (kosmetische) Mittel können beispielsweise als alkoholische oder wässrig-alkoholische Lösung, Lotion, Emulsion (Creme, Salbe), Gel etc. vorliegen und beispielsweise als Haarbehandlungsmittel, Schaumbad, Duschbad, Stiftpräparat, Deodorans, Sonnenschutzmittel etc. formuliert sein. Diese Mittel können ferner als Hilfs- und Zusatzstoffe Tenside, Ölkörper, Emulgatoren, Überfettungsmittel, Perlglanzwachse, Konsistenzgeber, Verdickungsmittel, Polymere, Silikonverbindungen, Fette, Wachse, Stabilisatoren, biogene Wirkstoffe, Deowirkstoffe, Antischuppenmittel, Filmbildner, Quellmittel, UV-Lichtschutzfaktoren, Antioxidantien, hydrotrope Stoffe, Konservierungsmittel, Insektenrepelentien, Selbstbräuner, Solubilisatoren, Parfümöle, Farbstoffe, keimhemmende Mittel und dergleichen enthalten.

Der erfindungsgemäße Extrakt ist in den Mitteln üblicherweise in einer Menge im Bereich von 0,01 bis 5 Gew.-%, vorzugsweise 0,1 bis 1 Gew.-%, bezogen auf das Gesamtgewicht des Mittels, enthalten. Der Extrakt wird im allgemeinen in einer Dosis von etwa 0,5 mg bis 100 mg/kg Körpergewicht pro Tag verabreicht. Er kann in einer Einzeldosis oder in mehreren Dosen gegeben werden.

Die Wirkung des Extraktes wurde anhand des nachstehenden Testsystems untersucht, wobei die Aktivität eines erfindungsgemäßen Extrakts in vitro im Vergleich zu der Aktivität eines handelsüblichen durch Extraktion mit wässrigem Ethanol erhaltenen Weidenrindenextraktes anhand der Cox-2-Hemmung bestimmt wurde. Als Messparameter wurde die $PGE_2$-Konzentration verwendet, die aufgrund der Hemmung durch Cox-2 verringert wird. Der Test wurde wie folgt durchgeführt:

Reagenzien:

DPBS-Puffer (0,10 g $KH_2PO_4$; 0,575 g $Na_2HPO_4$; 0,10 g KCl; 4,0 g NaCl; 0,50 g D-Glc; Wasser ad 500,0 ml).

Cremophor EL (Glycerin-Polyethylenglykolrhizinoleat)/EthanolLösung (116,9 g Cremophor EL; 50,0 g Ethanol).

Gentamycin/DPBS-Pufferlösung (25 mg Gentamycin-Sulfat; DPBS-Puffer ad 100,0 ml).

10 % Cremophor EL/Ethanol-Lösung (5 ml Cremophor EL/Ethanol; Gentamycin DPBS-Pufferlösung ad 50 ml).

ASS (Acetylsalicylsäure)-Lösung (ASS 2,0 mg; DPBS/Gentamycin-Lösung ad 10,0 ml).

LPS (Lipopolysaccharid) -Lösung (1,0 mg LPS, E. coli, serotyp 026: B6); DPBS/Gentamycin-Lösung ad 5,0 ml;

Thromboxan-Synthase-Hemmstoff (TSH) (1,4 mg TSH; Cremophor EL/Ethanol ad 5,0 ml);

0,5 ml dieser Lösung werden mit Gentamycin/DPBS-Puffer auf 5,0 ml aufgefüllt.

Für die Tests wird eine Stammlösung mit einer Konzentration von 300 mg/ml Weidenextrakt hergestellt durch Auffüllen von 3,0 g Weidenextrakt mit 10%iger Cremophor/Ethanol-Lösung auf 10 ml. Die Verdünnung von 30 mg/ml wird durch Verdünnen mit 10%igem Cremophor EL/Ethanol hergestellt.

Jeweils 800 µl Vollblut eines Spenders wird in Caps vorgelegt. In jedes Cap gibt man 10 µl TSH-Lösung und 50 µl ASS-Lösung. Für den Basalwert und für die Stimulationskontrollen werden 100 µl 10%iges Cremophor EL/Ethanol zugegeben. In die Caps mit Testverbindungen werden jeweils 100 µl der Testsubstanz gegeben.

Nach 15 minütiger Präinkubation im $CO_2$-Inkubator (37 °C, 5 % $CO_2$, 95 % feuchtigkeitsgesättigte Luft) gibt man in die Caps mit Testverbindungen und Stimulationskontrollen jeweils 50 µl LPS-Lösung, in die Basalwertproben 50 µl Gentamycin/DPBS-Pufferlösung. Zur Induktion der Cox-2-Proteinbiosynthese wird 5 h im $CO_2$-Inkubator unter obigen Bedingungen inkubiert. Anschließend kühlt man die Caps ab, gibt 1000 µl kalten DPBS-Puffer zu und zentrifugiert. Aus dem Überstand wird die $PGE_2$-Konzentration mittels ELISA bei einer Verdünnung von 1:20 bestimmt und die prozentuale Hemmung berechnet. Die erhaltenen Ergebnisse sind in der nachfolgenden Tabelle zusammengestellt.

Tabelle:

| Cox-2-Hemmung durch Weidenrindenextrakte | | |
|---|---|---|
| **Testsubstanz** | **300 mg/l** | **30 mg/l** |
| Handelsüblicher, wässrig/ethanolischer Weidenrindenextrakt | 43,3 % Hemmung | 3,8 % Hemmung |
| Erfindungsgemäßer $CO_2$-Weidenrinden-Extrakt gemäß Beispiel 3 | 54,8 % Hemmung | 24,9 % Hemmung |

Es ist ersichtlich, dass der erfindungsgemäße Extrakt Cyclooxygenase-2(Cox-2) überraschenderweise stärker hemmt und daher höhere Wirkung besitzt, als der Extrakt des Standes der Technik.

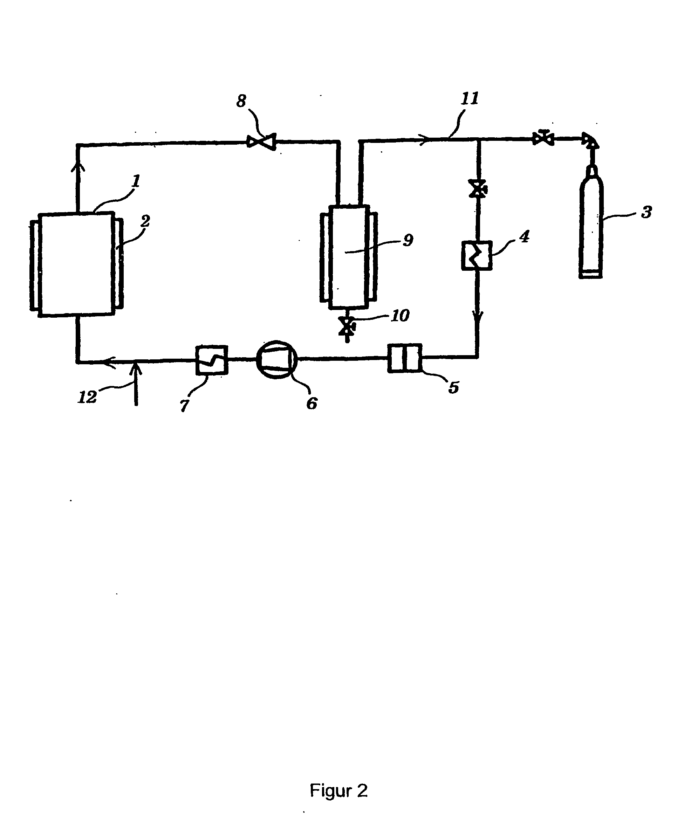

Die Gewinnung des erfindungsgemäßen Extraktes wird nachfolgend unter Bezug auf die in Figur 2 gezeigte Vorrichtung erläutert.

Figur 2 zeigt eine schematische Darstellung einer Vorrichtung, die zur Gewinnung des Extraktes brauchbar ist. Die zu extrahierenden Pflanzenteile befinden sich im Extraktionsbehälter (1), der mit einem Mantel (2) zum Durchleiten von Kühl- oder Wärmemedium ausgerüstet ist. Das zur Extraktion verwendete Kohlendioxid wird aus einem Vorratsbehälter (3) entnommen, über einen Wärmetauscher (4) und durch einen Filter (5) geführt und mit Hilfe einer Pumpe oder eines Kompressors (6) auf den gewünschten Extraktionsdruck gebracht. Der Wärmetauscher (7) dient dazu, die gewünschte Extraktionstemperatur einzustellen. Das Co-Solvens wird über Leitung (12) eingespeist. Das zur Extraktion verwendete Lösungsmittelgemisch durchströmt dann die im Extraktionsbehälter (1) befindlichen Pflanzenteile und verlässt mit Extraktstoffen beladen den Extraktionsbehälter (1). Am Ventil (8) erfolgt eine Entspannung auf den Abscheidedruck, gleichzeitig wird über das Ventil (8) die Durchflussrate geregelt. Der Extrakt scheidet sich zusammen mit dem Co-Solvens im Abscheidebehälter (9) ab und kann über das Ventil (10) entnommen werden. Im allgemeinen wird der Extrakt dann durch Abziehen des Co-Solvens oder Einengen und Filtration gewonnen. Anschließend erfolgt in der Regel eine Trocknung unter den oben angegebenen Bedingungen. Das Lösungsmittelgemisch wird über die Leitung (11) dem Kreislauf wieder zugeführt.

Beispiel 1

320 g einer auf eine Teilchengröße von weniger als 4 mm zerkleinerten Weidenrinde wurden in einem 2-Liter-Extraktionsbehälter einer Anlage gemäß Figur 2 mit einem Gemisch aus überkritischem Kohlendioxid und Ethanol extrahiert. Die Extraktionsbedingungen waren wie folgt:

Extraktionsdruck: 100 bar
Extraktionstemperatur: 60 °C
Abscheidedruck: 40 bar
Durchflussrate überkritisches Kohlendioxid: 7 kg/Stunde
Cosolvens: Ethanol 99,9 % mit 1 1/Stunde
Extraktionszeit: 2 Stunden
Trocknungsbedingungen: 45 °C/3 Tage bei Normaldruck

Salicingehalt des getrockneten Extrakts: 38 Gew.-%

Der getrocknete grobstückige Extrakt hat eine harzartige bis wachsartige feste Konsistenz.

Beispiel 2

Das Beispiel 1 wurde mit folgenden Extraktionsbedingungen wiederholt:

Extraktionsdruck: 100 bar
Extraktionstemperatur: 60 °C
Abscheidedruck: 40 bar
Durchflussrate überkritisches Kohlendioxid: 7 kg/Stunde
Cosolvens: Ethanol 99,9 % mit 1,5 1/Stunde
Extraktionszeit: 1,3 Stunden
Trocknungsbedingungen: 45 °C/3 Tage unter Vakuum
Salicingehalt des getrockneten Extrakts: 37 Gew.-%

Der getrocknete Extrakt ist pulvrig bis granulatartig, fließfähig und kann direkt zu Tabletten weiterverarbeitet werden.

Beispiel 3

930 g einer auf eine Teilchengröße von weniger als 4 mm zerkleinerten Weidenrinde wurden in einem 5-Liter-Extraktionsbehälter einer Anlage gemäß Figur 2 mit einem Gemisch aus überkritischem Kohlendioxid und Ethanol extrahiert. Die Extraktionsbedingungen waren wie folgt:

Extraktionsdruck: 100 bar
Extraktionstemperatur: 70 °C
Abscheidedruck: 40 bar
Durchflussrate überkritisches Kohlendioxid: 7 kg/Stunde
Cosolvens: Ethanol 99,9 % mit 1 1/Stunde
Extraktionszeit: 5 Stunden
Trocknungsbedingungen: 45 °C/3 Tage unter Vakuum
Salicingehalt des getrockneten Extrakts: 42 Gew.-%

Der getrocknete Extrakt ist pulvrig bis granulatartig und kann direkt zu Tabletten weiterverarbeitet werden.
Der gemäß Beispiel 3 erhaltene Extrakt wurde dünnschichtchromatographisch untersucht. Zum Vergleich wurde auch ein handelsüblicher Extrakt (Dragenopharm 1581) und eine Vergleichslösung, die Glukose, Salicin und Cyanidol enthielt, untersucht. Die Dünnschichtchromatographie wurde folgendermaßen durchgeführt:

200 mg des trockenen Extraktes wurden in 10 ml Methanol bei 60 °C gelöst. Die Lösung wurde filtriert und mit 0,5 g Polyamidpulver unter Schütteln behandelt. Das Polyamid wurde abfiltriert und 2 x mit 3 ml Methanol gewaschen. Das Filtrat wurde unter verringertem Druck an einem Rotationsverdampfer bei einer Wasserbadtemperatur von nicht mehr als 50 °C zur Trockene eingeengt und der Rückstand wurde in 3 ml Methanol gelöst. 10 µl dieser Lösung (Testlösung A) wurden dünnschichtchromatographisch aufgetrennt.

Da der Extrakt auch Salicinester und andere Ester enthält, wurde eine weitere Probe des Extraktes wie oben beschrieben bis zum Verdampfen des Filtrats behandelt. Das Verdampfen des Filtrats erfolgte jedoch nicht bis zur Trockene, sondern bis zu einem Volumen von etwa 5 ml. Dazu gab man 5 ml 0,2 N-Natriumhydroxidlösung und erhitzte 60 Min in einem Wasserbad unter häufigem Schütteln auf 60 °C. Nach dem Abkühlen gab man 1 ml 1N-Salzsäurelösung zu, verdampfte zur Trockene unter vermindertem Druck bei einer Wasserbadtemperatur von nicht mehr als 50 °C und löste den Rückstand in 3 ml Methanol, 10 µl dieser Lösung wurden dünnschichtchromatographisch untersucht (Testlösung B).
Zur Herstellung der Vergleichslösung wurden 2,0 mg Salicin, 5,0 mg Cianidanol und 5,0 mg Glukose in 1,0 ml Methanol gelöst. 10 µl dieser Lösung wurden dünnschichtchromatographisch untersucht.
Laufmittel: Wasser-wasserfreie Ameisensäure-Ethylacetat im Volumenverhältnis von 7:13:80.
Dünnschichtchromatographie-Platten: vorbeschichtetes Kieselgur 60 $F_{254}$ der Firma Merck, Schichtdicke 250 µm auf 20 x 20 cm Glasplatten.

Zur Detektion wurde ein Sprühreagens aus 5 ml Schwefelsäure 96 % und 95 ml einer 0,5 %igen (G/V) Lösung von Thymol in 96 %igem Ethanol verwendet.

Laufmittelstrecke: 12 cm von der Auftragslinie bis zur Lösungsmittelfront.

Nach der Entwicklung wurden die Platten aus der Kammer genommen und 5 Minuten bei 100 bis 105 °C getrocknet. Nach dem Abkühlen wurden sie besprüht, 10 bis 15 Minuten auf 120 °C erhitzt und ausgewertet.

Mit den Testlösungen A wurden die Chromatogramme 1 und 4, mit den Testlösungen B die Chromatogramme 2 und 5 und mit der Vergleichslösung das Chromatogramm 3 erhalten.

Die quantitative Bestimmung des Salicingehaltes erfolgte mittels HPLC wie folgt:

Als Referenzprobe wurde eine Lösung von 25 mg Salicin in 100 ml destilliertem Wasser verwendet. Für die Testlösung wurden 50 mg Weidenextrakt in 5 ml Wasser und 5 ml 0,2 N Natriumhydroxidlösung gelöst und 60 Minuten bei 60 °C behandelt. Nach dem Abkühlen wurde mit 1 N HCl auf den pH der Referenzlösung eingestellt und mit Wasser auf 100 ml aufgefüllt. Nach Filtration durch einen 0,2 $\mu$m Celluloseacetatfilter erfolgte die Gehaltsbestimmung unter folgenden Bedingungen:

Säule: 250 x 4 mm innerer Durchmesser (Fa. Merck KGaA, 64271 Darmstadt, Deutschland;
Stationäre Phase: LiChrosorb Si 60 RP-18, 10 $\mu$m (Fa. Merck);
Mobile Phase: Wasser mit 25 % V/V Tetrahydrofuran;
Fließrate: 1,0 ml/min;
Probe: 20 $\mu$l;
UV-Detektion: bei 213 nm;
Absorbtionsbereich: 0,08;
Berechnung des prozentualen Salicingehaltes $S_c$ (G/G):

$$S_c = \frac{C_1 \cdot A_2}{C_2 \cdot A_1} \cdot 100$$

$C_1$ = Konzentration der Referenzlösung in mg/100 ml.
$C_2$ = Konzentration des Extraktes in der Testlösung in mg/100 ml
$A_1$ = Fläche des Salicinpeaks im Chromatogramm der Referenzlösung
$A_2$ = Fläche des Salicinpeaks im Chromatogramm der Testlösung.

**Patentansprüche**

1. Weidenrindenextrakt, enthaltend wenigstens 25 Gew.-% Salicin, bezogen auf den Feststoffgehalt und bestimmt nach alkalischer Verseifung.

2. Weidenrindenextrakt nach Anspruch 1, enthaltend 30 bis 50 Gew.-% Salicin.

3. Weidenrindenextrakt nach Anspruch 1, enthaltend 35 bis 45 Gew.-% Salicin.

4. Weidenrindenextrakt, erhältlich indem man zerkleinerte entlaubte Zweige, Zweigspitzen und/oder Rinde der Weide mit einem Lösungsmittelgemisch aus einem überkritischen Gas und mindestens einem organischen Co-Solvens extrahiert und den erhaltenen Flüssigextrakt von dem Lösungsmittelgemisch befreit.

5. Weidenrindenextrakt nach Anspruch 4, wobei man als überkritisches Gas Kohlendioxid verwendet.

6. Weidenrindenextrakt nach einem der vorhergehenden Ansprüche in Form eines Trockenpulvers.

7. Verfahren zur Herstellung des Weidenrindenextraktes nach einem der Ansprüche 1 bis 6, wobei man zerkleinerte entlaubte Zweige, Zweigspitzen und/oder Rinde der Weide mit einem Lösungsmittelgemisch aus einem überkritischen Gas und mindestens einem organischen Co-Solvens extrahiert und den erhaltenen Flüssigextrakt von dem Lösungsmittelgemisch befreit.

8. Verfahren nach Anspruch 7, wobei man als überkritisches Gas Kohlendioxid verwendet.

9. Verfahren nach Anspruch 7 oder 8, wobei man die noch vorhandenen flüchtigen Bestandteile durch Vakuumtrocknung oder Gefriertrocknung entfernt.

10. Pharmazeutisches oder kosmetisches Mittel, enthaltend einen Weidenrindenextrakt nach einem der Ansprüche 1 bis 6.

11. Pharmazeutisches Mittel nach Anspruch 10 in Form einer oralen Dosierungsform.

12. Pharmazeutisches oder kosmetisches Mittel nach Anspruch 10 in Form einer topischen Zubereitung.

13. Verwendung eines Weidenrindenextraktes nach einem der Ansprüche 1 bis 6 zur Herstellung eines pharmazeutischen Mittels zur Behandlung von Erkrankungen des rheumatischen Formenkreises oder zur präventiven oder therapeutischen Behandlung von allergisch induzierten Erkrankungen.

## Claims

1. Willow bark extract, containing at least 25 wt% salicin, based on the solids content and determined by alkaline saponification.

2. Willow bark extract according to claim 1, containing 30 to 50 wt% salicin.

3. Willow bark extract according to claim 1, containing 35 to 45 wt% salicin.

4. Willow bark extract, obtainable by extracting comminuted defoliated willow twigs, twig tips and/or bark with a solvent mixture of a supercritical gas and at least one organic co-solvent, and freeing the obtained liquid extract of the solvent mixture.

5. Willow bark extract according to claim 4, whereby the supercritical gas used is carbon dioxide.

6. Willow bark extract according to one of the preceding claims in the form of a dry powder.

7. Process for the preparation of the willow bark extract according to one of claims 1 to 6, whereby comminuted defoliated willow twigs, twig tips and/or bark are extracted with a solvent mixture of a supercritical gas and at least one organic co-solvent, and the obtained liquid extract is freed of the solvent mixture.

8. Process according to claim 7, whereby the supercritical gas used is carbon dioxide.

9. Process according to claim 7 or 8, whereby the remaining volatile components are removed by vacuum- or freeze-drying.

10. Medicament or cosmetic agent, containing a willow bark extract according to one of claims 1 to 6.

11. Medicament according to claim 10 in the form of an oral dosage form.

12. Medicament or cosmetic agent according to claim 10 in the form of a topical preparation.

13. Use of a willow bark extract according to one of claims 1 to 6 in the preparation of a medicament for the treatment of rheumatic diseases or for the preventive or therapeutic treatment of allergically induced illnesses.

## Revendications

1. Extrait d'écorce de saule blanc, contenant au moins 25 % en poids de salicine, par rapport à la teneur en solides et tel que déterminé après saponification alcaline.

2. Extrait d'écorce de saule blanc selon la revendication 1, contenant de 30 à 50 % en poids de salicine.

**3.** Extrait d'écorce de saule blanc selon la revendication 1, contenant de 35 à 45 % en poids de salicine.

**4.** Extrait d'écorce de saule blanc obtenu en extrayant des branches, des terminaisons de branches effeuillées et/ou d'écorce de saule blanc broyées à l'aide d'un mélange solvant composé d'un gaz supercritique et d'au moins un co-solvant organique et en enlevant l'extrait liquide produit du mélange solvant.

**5.** Extrait d'écorce de saule blanc selon la revendication 4, dans lequel le gaz supercritique utilisé est le dioxyde de carbone.

**6.** Extrait d'écorce de saule blanc selon l'une quelconque des revendications précédentes sous la forme d'une poudre sèche.

**7.** Procédé de préparation de l'extrait d'écorce de saule blanc selon les revendications 1 à 6, dans lequel on extrait des branches, des terminaisons de branches effeuillées et/ou de l'écorce de saule blanc broyées à l'aide d'un mélange solvant composé d'un gaz supercritique et d'au moins un co-solvant organique et on enlève l'extrait liquide produit du mélange solvant.

**8.** Procédé selon la revendication 7, dans lequel le gaz supercritique utilisé est le dioxyde de carbone.

**9.** Procédé selon la revendication 7 ou 8, dans lequel on élimine les composants volatils encore présents par séchage sous vide ou lyophilisation.

**10.** Produit pharmaceutique ou cosmétique, contenant un extrait d'écorce de saule blanc selon l'une quelconque des revendications 1 à 6.

**11.** Produit pharmaceutique selon la revendication 10 sous la forme d'une forme posologique orale.

**12.** Produit pharmaceutique ou cosmétique selon la revendication 10, sous la forme d'une préparation topique.

**13.** Utilisation d'un extrait d'écorce de saule blanc selon l'une quelconque des revendications 1 à 6 pour la préparation d'un produit pharmaceutique permettant le traitement des affections rhumatismales ou le traitement préventif ou thérapeutique des troubles allergiques.

Figur 1

Figur 2